# EUROPÄISCHE PATENTANMELDUNG

(11) **EP 1 231 202 A2**
(43) Veröffentlichungstag der Anmeldung: **14.08.2002**
(21) Anmeldenummer: 02002118.4
(22) Anmeldetag: 29.01.2002
(51) Int. Cl.: C07C 67/03, C07C 69/54, C07D 233/32

(54) **Umesterung von (Meth)acrylsäureestern in Gegenwart von Alkalimetallcyanat enthaltenden Mischkatalysatoren**

(30) Priorität: 12.02.2001 DE 10106642
(71) Anmelder: Röhm GmbH & Co. KG, 64293 Darmstadt (DE)
(72) Erfinder: Knebel, Joachim, Dr., 64665 Alsbach-Hähnlein (DE); Merbach, Ralf, 64572 Büttelborn (DE); Nuccio, Santa-Marina, 64331 Weiterstadt (DE)

(57) **Zusammenfassung**

Es wird ein Verfahren zur Herstellung von (Meth)acrylsäureestern durch Umesterung beschrieben, bei dem man als Katalysator ein Gemisch aus einem Alkalimetallcyanat oder Alkalimetallthiocyanat und einem Erdalkalimetalloxid, einem Erdalkalimetallhydroxid oder einem Alkalimetallhalogenid verwendet.

## Beschreibung

### Gebiet der Erfindung

Die Erfindung betrifft ein Verfahren zur Herstellung von Estern der Acryl- und der Methacrylsäure, insbesondere Ester von höheren Alkoholen und substituierten Alkoholen, ausgehend von großtechnisch einfach zugänglichen Estern der Acryl- bzw. Methacrylsäure mit Alkohol durch Umesterung unter Verwendung von Alkalimetallcyanaten enthaltenden Mischkatalysatoren.

### Stand der Technik

Methacrylsäurester oder Acrylsäureester erhält man üblicherweise durch Umsetzung von Alkoholen mit einfachen Methacrylsäureestern wie beispielsweise Methylmethacrylat oder Ethylacrylat. Dazu kommen alkalische Katalysatoren wie zum Beispiel Lithium- und Calciumhydroxide zum Einsatz, oder man arbeitet mit Metallkatalysatoren wie Titanalkoholaten oder auch Organo-Zinnverbindungen (DE 3423441, 3423443, Röhm GmbH). Hinsichtlich ihrer Abtrennbarkeit sind alkalische Katalysatoren vorteilhaft, da sie ohne vorhergehende Trennprozesse wie Filtration beseitigt werden können. Ihr Lithiumanteil bestimmt im wesentlichen ihren Preis, so das preisgünstige Katalysatorkomponenten wünschenswert wären.

Als selektive Umesterungskatalysatoren sind auch Alkalimetallcyanide bekannt (Houben-Weyl, Methoden der organischen Chemie, Band 5, Seite 702-703, Thieme Verlag 1985). Ihre Toxizität beschränkt allerdings ihre industriellen Einsatzmöglichkeiten. Die weniger giftigen Alkalimetallcyanate eignen sich nur in Sonderfällen zur Umesterungskatalyse, nämlich dann, wenn man reaktive Substrate wie Glycidol oder Kohlensäureester einsetzt.

So beschreibt die DE 25 25 026 (Degussa AG) die Herstellung von Glycidylmethacrylat durch Umesterung von Methylmethacrylat mit Glycidol in Gegenwart eines Umesterungskatalysators und eines Polymerisationsinhibitors. Als Umesterungskatalysator werden Kaliumcyanid, Kaliumcyanat und Kaliumthiocyanat verwendet.

JP 55094380 beschreibt ebenfalls ein Verfahren zur Herstellung von Glycidylmethacrylat durch Umesterung in Gegenwart von Kaliumcyanat. Die Ausbeute an Glycidylmethacrylat beträgt 83,5 %.

EP 683 163 beschreibt die Herstellung von Glycidyl(meth)acrylat durch Umesterung von Methyl(meth)acrylaten in Gegenwart eines Polymerisationsinhibitors und eines Katalysators. Der Katalysator wird in situ aus einem Ammoniumsalz oder Phosphoniumhalogenid und Kaliumcyanid, -cyanat oder -thiocyanat hergestellt. Am Ende der Reaktion ist ein Desaktivierungsschritt des Katalysators notwenig, der darin besteht, Alkalioder Erdalkalimetallsalze einer Sulphonsäure oder einer Heteropolysäure zum Reaktionsgemisch zuzugeben.

### Aufgabe

Es bestand also die Aufgabe, ein Verfahren zur Herstellung von Methacrylsäureestern durch Umesterung in Gegenwart eines Mischkatalysatorsystems zu finden, dass die Nachteile des Standes der Technik, wie beispielsweise die Verwendung von giftigen Alkalimetallcyaniden oder die Notwendigkeit zusätzlicher Raktionsschritte oder Stoffzugaben zum Deaktivieren des Katalysators vermeidet, einfach durchführbar ist und in dem keine speziellen Desaktivierungsschritte notwendig sind, die auf Kosten der Ausbeute an Spezialester gehen. Für die technische Durchführbarkeit des Verfahrens ist es weiter wichtig, dass der Katalysator nach erfolgter Reaktion leicht aus dem Rohester abgetrennt werden kann.

### Lösung

Es wurde nun gefunden, dass in Gegenwart von Mischungen aus Alkalimetallcyanaten oder Alkalimetallthiocyanaten und Alkali- oder Erdalkalimetallsalzen Alkohole zu den entsprechenden Methacrylsäureestern mit hoher Reinheit und mit wenig Nebenprodukten umgesetzt werden können. Unter Alkalimetallcyanaten wird beispielsweise Natriumcyanat oder Kaliumcyanat verstanden, unter Alkalimetallthiocyanaten werden beispielsweise Natriumthiocyanat oder Kaliumthiocyanat verstanden.

Die Alkalimetallcyanate oder die Alkalimetallthiocyanate können einzeln oder in Mischungen eingesetzt werden. Als Alkalimetallsalz wird ein Alkalimetallhalogenid, beispielsweise Natriumchlorid, Kaliumchlorid, Natriumfluorid, Kaliumfluorid, Natriumbromid, Kaliumbromid, Natriumiodid, Lithiumchlorid, Lithiumbromid oder Kaliumiodid angewandt.

Als Erdalkalimetallsalz wird ein Erdalkalimetalloxid, wie beispielsweise Magnesiumoxid, Calciumoxid oder Bariumoxid angewandt. Es können auch die entsprechenden Hydroxide eingesetzt werden.

Für die technische Durchführbarkeit des Verfahrens ist es vorteilhaft, dass der Katalysator nach erfolgter Reaktion leicht und ohne zusätzliche Verfahrensschritte aus dem Rohester abgetrennt werden kann. Dies gelingt besonders einfach durch Filtration. Ein weiterer Vorteil gegenüber den bekannten Titankatalysatoren besteht aus der Wassertoleranz des Katalysatorsystems. Wasser im Reaktionssystem deaktiviert sofort die Titankatalysatoren.

### Beispiele

### Verwendete Apparatur:

In eine Apparatur, bestehend aus einem 0,51 Dreihalskolben mit mechanischem Rührer, Lufteinleitung und aufgesetzter Füllkörperkolonne (30 cm lang, gefüllt mit 6 mm Raschigringen) sowie Kolonnenkopf mit Rücklaufteiler werden die Komponenten, bestehend aus dem umzuesternden Alkohol und Methylmethacrylat, eingefüllt. Als Stabilisierung gegen vorzeitige Polymerisation dient Hydrochinonmonomethylether, ggf. werden weitere Inhibitoren hinzugesetzt.

### Allgemeine Arbeitsvorschrift für die Umesterung

Unter Lufteinleiten wird zunächst bis zum Sieden erhitzt und das bei nassen Einsatzstoffen sich bildende Methylmethacrylat-Wasser-Aceotrop über die Kolonne so lange abdestilliert, bis die Kopftemperatur konstant bei 100 °C liegt und ein klares MMA übergeht. Dann lässt man auf ca. 90 °C Sumpftemperatur abkühlen, ergänzt die durch die Destillation entfernte MMA-Menge durch reines MMA, gibt den Katalysator hinzu und erhitzt erneut zum Sieden, wobei das gebildete MMA-Methanol-Aceotrop übergeht (Kopftemperatur: 65 - 99 °C). Gegen Ende der Reaktion nähert sich die Kopftemperatur dem Siedepunkt des reinen MMA (100 °C bei Normaldruck). Wenn nur noch reines MMA übergeht, ist die Umesterung beendet, man läßt abkühlen und zieht im Vakuum (ca. 10 mbar) das restliche MMA ab, den sich bildenden Katalysatorniederschlag trennt man durch Filtration ab. Das Filtrat wird durch Gaschromatographie analysiert.

### Beispiel 1

Einsatzstoffe sind 32,5 g Ethylenglycol und 300 g MMA sowie 6,65 g (2% bez. auf Einsatzstoffe) Kaliumcyanat sowie 1 g Calciumoxid. Stabilisiert wird mit 71 ppm Hydrochinonmonomethylether, 38 ppm N,N'-Diphenyl-pphenylendiamin und 9 ppm 4-Hydroxy-2,2,6,6-tetramethylpiperidyl-N-oxyl (bez. auf die Summe Eingangsstoffe).
Reaktionszeit: 5,5 h, Ausbeute: 100 g
Zusammensetzung des Endprodukts nach GC: 94,3 %
Ethylenglycoldimethacrylat, 0,53% Hydroxyethylmethacrylat, 4,2 % Additionsprodukte.

### Beispiel 2

Einsatzstoffe sind 31 g Ethylenglycol und 300 g MMA sowie 2 g Natriumcyanat und 1,3 g Lithiumchlorid (zusammen 1% bez. auf Einsatzstoffe). Stabilisiert wird mit 71 ppm Hydrochinonmonomethylether, 38 ppm N,N'-Diphenyl-p-phenylendiamin und 9 ppm 4-Hydroxy-2,2,6,6-tetramethylpiperidyl-N-oxyl (bez. auf Eingangsstoffe). Das MMA-haltige Reaktionsgemisch wird filtriert, MMA wird nicht abgezogen.

Reaktionszeit: 4,5 h, Ausbeute: 210 g (enthält 47,5 % MMA)
Zusammensetzung des Endprodukts nach GC (MMA herausgerechnet): 93,3 % Ethylenglycoldimethacrylat, 6,7% Hydroxyethylmethacrylat.

### Vergleichsbeispiel 3

### (Vergleichsbeispiel mit Kaliumcyanat als alleinigem Katalysator)

Einsatzstoffe sind 31 g Ethylenglycol und 300 g MMA sowie 3,3g (1% bez. auf Einsatzstoffe) Kaliumcyanat. Stabilisiert wird mit 71 ppm Hydrochinonmonomethylether, 38 ppm N,N'-Diphenyl-p-phenylendiamin und 9 ppm 4-Hydroxy-2,2,6,6-tetramethylpiperidyl-N-oxyl (bez. auf Eingangsstoffe). Das MMA-haltige Reaktionsgemisch wird filtriert, MMA wird nicht abgezogen.

Reaktionszeit: 4h, Ausbeute: 313 g (enthält 68,3 % MMA)
Zusammensetzung des Endprodukts nach GC (MMA herausgerechnet): 62 % Ethylenglycoldimethacrylat, 33 % Hydroxyethylmethacrylat Kaliumcyanat allein führt zu unvollständigem Umsatz.

### Beispiel 4

### Herstellung von Ethylenglycoldimethacrylat, Vergleichsbeispiel mit Calciumoxid als alleinigem Katalysator

Einsatzstoffe sind 31 g Ethylenglycol und 300 g MMA sowie 3,3g (1% bez. auf Einsatzstoffe) Calciumoxid. Stabilisiert wird mit 71 ppm Hydrochinonmonomethylether, 38 ppm N,N'-Diphenyl-p-phenylendiamin und 9 ppm 4-Hydroxy-2,2,6,6-tetramethylpiperidyl-N-oxyl (bez. auf Eingangsstoffe). Das MMA-haltige Reaktionsgemisch wird filtriert, MMA wird nicht abgezogen.
Reaktionszeit: 4h, Ausbeute: 339 g (enthält 73,9 % MMA)
Zusammensetzung des Endprodukts nach GC (MMA herausgerechnet): 44,7 % Ethylenglycoldimethacrylat, 50,3 % Hydroxyethylmethacrylat Calciumoxid allein führt zu unvollständigem Umsatz.

### Vergleichsbeispiel 5

### Herstellung von N-(Methacryloyloxyethyl)-2-imidazolidinon, Vergleichsbeispiel mit Kaliumcyanat als alleinigem Katalysator

### Apparatur:

1 I-Wittscher Topf, Blattrührung, Lufteinleitrohr, 50-cm-Kolonne NS45 mit 8 x 8 mm Raschigringen, Kolonnenkopf (Flüssigteiler), Rückfluß- und Produktkühler, Vorlage, Ölumlaufthermostat zur Beheizung,
Temperaturmessung im Sumpf und Kolonnenkopf, Steuergerät DEST-Star 2 und Contact-Celsometer (beide Fa. NGW) zur Steuerung des Destillatabzugs.

### Versuchsdurchführung:

Die Einwaage wird ohne Katalysator unter Rühren und Einleiten von wenig Luft zum Sieden aufgeheizt und evtl. vorhandenes Wasser azeotrop mit MMA abdestilliert. Danach wird ca. 10 °C abgekühlt, der Katalysator und die dem Azeotropdestillat äquivalente Masse MMA zugefügt und aufgeheizt. Das entstehende MMA/Methanol-Aceotrop wird bei einem Rücklaufverhältnis von 5:1 und einer Kopftemperaturbegrenzung von 70 °C abdestilliert, bis die Temperatur nicht mehr unter den eingestellten Grenzwert fällt. Jetzt wird Kopftemperatur schrittweise bis 100 °C angehoben bis zum Alkoholyseende nur noch MMA siedet.

Es wird 10 Minuten ohne Destillatabzug weiter refluxiert, um sicherzustellen, daß die Alkoholyse tatsächlich beendet ist (Kopftemperaturkonstanz). Der Rohester wird dann auf Raumtemperatur abgekühlt und nach Abtrennung des ungelösten Katalysators über einen Faltenfilter wird das klare Filtrat gaschromatographisch analysiert.

Einsatzstoffe sind 260 g N-(Hydroxyethyl)-2-imidazolidinon und 800 g MMA sowie 2,6g (1% bez. auf Imidazolidinon) Kaliumcyanat. Stabilisiert wird mit 0,7 g Hydrochinonmonomethylether, 0,24 g Phenothiazin und 0,012 g 4-Hydroxy-2,2,6,6-tetramethylpiperidyl-N-oxyl.
Reaktionszeit: 8h Ausbeute: 823 g
Zusammensetzung: 56 % MMA, 9% unumgesetztes N-(Hydroxyethyl)-2-imidazolidinon, 32 % N-(Methacryloyloxyethyl)-2-imidazolidinon, 0,3 % N-(Methacryloyloxyethyl)-N'-(methacryloyl)-2-imidazolidinon, 1% N-(Methacryloyloxyethyl)-N'-(2-(methoxycarbonyl)propyl)-2-imidazolidinon.

Die Umesterung ist unvollständig.

### Beispiel 6

### Herstellung von N-(Methacryloyloxyethyl)-2-imidazolidinon

### Versuchsdurchführung wie in Beispiel 5

Einsatzstoffe sind 260 g N-(Hydroxyethyl)-2-imidazolidinon und 800 g MMA sowie 2,54g Kaliumcyanat und 0,065 g Calciumhydroxid ( zusammen 1% bez. auf Imidazolidinon). Stabilisiert wird mit 0,7 g Hydrochinonmonomethylether, 0,24 g Phenothiazin und 0,012 g 4-Hydroxy-2,2,6,6-tetramethylpiperidyl-N-oxyl.
Reaktionszeit: 4,75 h, Ausbeute 820 g
Zusammensetzung: 52,6 % MMA, 1,5% unumgesetztes N-(Hydroxyethyl)-2-imidazolidinon, 38,7 % N-(Methacryloyloxyethyl)-2-imidazolidinon, 1,76 % N-(Methacryloyloxyethyl)-N'-(methacryloyl)-2-imidazolidinon, 3,17% N-(Methacryloyloxyethyl)-N'-(2-(methoxycarbonyl)propyl)-2-imidazolidinon.

## Patentansprüche

1. Verfahren zur Herstellung von Estern der Acrylsäure oder Methacrylsäure durch Umesterung der Acryl- oder Methacrylsäureester von C₁-C₄-Alkoholen mit davon verschiedenen Alkoholen in Gegenwart eines Katalysatorsystems,
**dadurch gekennzeichnet, dass**
man als Katalysatorsystem Alkalimetallcyanat oder Alkalimetallthiocyanat und Erdalkalimetalloxid oder Erdalkalimetallhydroxid oder Alkalimetallhalogenid verwendet.

2. Verfahren nach Anspruch 1
**dadurch gekennzeichnet, dass**
man als Katalysatorsystem ein Gemisch aus Alkalimetallcyanat und Erdalkalimetalloxid verwendet.

3. Verfahren nach Anspruch 1
**dadurch gekennzeichnet, dass**
man als Katalysatorsystem ein Gemisch aus Alkalimetallcyanat und Erdalkalimetallhydroxid verwendet.

4. Verfahren nach Anspruch 1
**dadurch gekennzeichnet, dass**
man als Katalysatorsystem ein Gemisch aus Alkalimetallcyanat und Alkalimetallsalz verwendet.

5. Verfahren nach Anspruch 1
**dadurch gekennzeichnet, dass**
man als Alkalimetallsalz Lithiumchlorid verwendet.

6. Verfahren nach einem der vorherigen Ansprüche
**dadurch gekennzeichnet, dass**
das Katalysatorsystem in Mengen von 0,5 - 5 %, bezogen auf die Reaktionsmischung, eingesetzt wird.

7. Verfahren nach einem der vorherigen Ansprüche
**dadurch gekennzeichnet, dass**
das Verhältnis zwischen Alkalimetallcyanat und Erdalkalimetalloxid zwischen 10:1 und 1:2 beträgt.

8. Verfahren nach einem der vorherigen Ansprüche
**dadurch gekennzeichnet, dass**
das Verhältnis zwischen Alkalimetallcyanat und Erdalkalimetallhydroxid zwischen 50:1 und 1:2 beträgt.

9. Verfahren nach einem der vorherigen Ansprüche
**dadurch gekennzeichnet, dass**
das Gewichtsverhältnis zwischen Alkalimetallcyanat und Alkalimetallhalogenid zwischen 5:1 und 1:1 besteht.
